# EUROPEAN PATENT SPECIFICATION

(11) **EP 1 608 294 B1**
(45) Date of publication and mention of the grant of the patent: **26.12.2007**
(21) Application number: 04758455.2
(22) Date of filing: 25.03.2004
(51) Int. Cl.: A61F 2/82

(54) **DETACHABLE AND RETRIEVABLE STENT ASSEMBLY**
ABNEHMBARE UND RÜCKHOLBARE STENTANORDNUNG
ENSEMBLE DRAIN TUTEUR AMOVIBLE ET RECUPERABLE

(30) Priority: 02.04.2003 US 459696 P
(43) Date of publication of application: 28.12.2005
(73) Proprietor: Boston Scientific Limited, St. Michael (BB)
(72) Inventor: BASHIRI, Mehran, San Carlos, CA 94070 (US); PHUNG, Mark, Union City, CA 94587 (US); RAMZIPOOR, Kamal, Fremont, CA 94539 (US); NAIR, Ajitkumar, B., Milpitas, CA 95035 (US)
(74) Representative: Hauck Patent- und Rechtsanwälte
(86) International application number: PCT/US2004/009406
(87) International publication number: WO 2004/087017

(56) References cited:
- EP-A- 0 938 878
- WO-A-94/03127
- US-A- 5 057 092

## Description

### BACKGROUND OF THE INVENTION

Stents, grafts, stent-grafts, vena cava filters and similar implantable medical devices, collectively referred to hereinafter as stents, are radially expandable endoprostheses which are typically intravascular implants capable of being implanted transluminally and enlarged radially after being introduced percutaneously. Stents may be implanted in a variety of body lumens or vessels such as within the vascular system, urinary tracts, bile ducts, etc. Stents may be used to reinforce body vessels and to prevent restenosis following angioplasty in the vascular system. They may be self-expanding, mechanically expandable or hybrid expandable.

Stents are generally tubular devices for insertion into body lumens. However, it should be noted that stents may be provided in a wide variety of sizes and shapes. Balloon expandable stents require mounting over a balloon, positioning, and inflation of the balloon to expand the stent radially outward. Self-expanding stents expand into place when unconstrained, without requiring assistance from a balloon. A self-expanding stent may be biased so as to expand upon release from the delivery catheter and/or include a shape-memory component which allows the stent to expand upon exposure to a predetermined condition. Some stents may be characterized as hybrid stents which have some characteristics of both self-expandable and balloon expandable stents.

Due to the branching nature of the human vasculature it is not uncommon for stenoses to form at any of a wide variety of vessel bifurcations. A bifurcation is an area of the vasculature or other portion of the body where a first (or parent) vessel is bifurcated into two or more branch vessels. In some cases it may be necessary to implant multiple stents at the bifurcation in order to address a stenosis located thereon. Alternatively, a stent may be provided with multiple sections or branches that may be deployed within the branching vessels of the bifurcation.

Stents may be constructed from a variety of materials such as stainless steel, Elgiloy, nickel, titanium, nitinol, shape memory polymers, etc. Stents may also be formed in a variety of manners as well. For example a stent may be formed by etching or cutting the stent pattern from a tube or sheet of stent material; a sheet of stent material may be cut or etched according to a desired stent pattern whereupon the sheet may be rolled or otherwise formed into the desired substantially tubular, bifurcated or other shape of the stent; one or more wires or ribbons of stent material may be woven, braided or otherwise formed into a desired shape and pattern. Stents may include components that are welded, bonded or otherwise engaged to one another.

Typically, a stent is implanted in a blood vessel or other body lumen at the site of a stenosis or aneurysm by so-called "minimally invasive techniques" in which the stent is compressed radially inwards and is delivered by a catheter to the site where it is required through the patient's skin or by a "cut down" technique in which the blood vessel concerned is exposed by minor surgical means. When the stent is positioned at the correct location, the stent is caused or allowed to expand to a predetermined diameter in the vessel.

Stents are currently utilized in a variety of applications. However, in some applications, such as for example in procedures involving intracranial placement of a stent, the delivery and placement of the stent is particularly challenging due considerations including access, visualization, control, etc. Many existing stents do not sufficiently address the need for exact placement and/or the need to reposition the stent within a lumen, as may be necessary in some intracranial procedures. Despite the wide variety of stents presently available, there remains a desire to provide stents and stent designs which provide a stent that is capable of precise placement, including the ability to reposition the stent following its expansion.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### BRIEF SUMMARY OF THE INVENTION

In light of the above the present invention is directed to a variety of embodiments. In at least one embodiment the invention comprises a stent that is repositionable within a body lumen even after the stent has been expanded.

In at least one embodiment the stent is self-expandable.

In at least one embodiment the stent is inflation expandable.

In at least one embodiment the stent is hybrid expandable.

In at least one embodiment the stent is constructed from a nickel titanium alloy such as nitinol.

In at least one embodiment the stent is at least partially constructed from a cut tube.

In at least one embodiment the stent is at least partially constructed from one or more wires.

In at least one embodiment the stent is configured to deliver one or more therapeutic agents.

In at least one embodiment the stent comprises one or more coatings.

In at least one embodiment the stent defines a back bone, the back bone is a longitudinal component of the stent having one or more physical characteristics different than the remainder of the stent. In some embodiments the back bone comprises a wall thickness that is greater than that of the wall thickness of the remainder of the stent. In some embodiments the variable thickness of the stent may be provided by masking selected portions of the stent prior to microblasting and/or electropolishing the stent. In some embodiments the back bone provides the stent with improved manipulation or 'push' characteristics by providing a portion of the stent with a longitudinal component that has a greater column strength than the remainder of the stent. In some embodiments the stent is provided with a plurality of back bone elements. In some embodiments the back bone has one or more components that are oriented in a direction other than in the longitudinal direction of the stent.

In at least one embodiment the stent is at least partially radiopaque. In some embodiments one or more radiopaque markers are engaged to the back bone or are positioned adjacent thereto.

In at least one embodiment the stent has a variable stiffness.

In at least one embodiment, prior to delivery of the stent, the stent is engaged to a push/pull wire, hereinafter referred to as a "push wire", the push wire being engaged to the stent at a proximal severable junction. In some embodiments the severable junction is non-conductive. In some embodiments the stent is released from the push wire when the severable junction is severed or otherwise disrupted. In some embodiments the severable junction is severed by electrolytic corrosion, mechanical actuation, application of hydraulic pressure, one or more thermal processes, application of electromagnetic energy, etc. The push wire is constructed and arranged to allow a user to manipulate the stent within a lumen even after the stent has been deployed and/or expanded. Once the stent is positioned in a manner and location that is desired the severable junction is severed and the push wire may be withdrawn.

In at least one embodiment the stent is characterized as comprising a plurality of struts that are moveable in a variety of directions when the stent expands. The moveable struts provide the stent with the capability of being folded, collapsed and/or expanded in a manner that aids in minimizing the level of strain on the individual struts. In some embodiments struts are moveable in a direction substantially perpendicular to the longitudinal axis of the stent. In some embodiments struts are moveable in such a manner so as to provide the stent with a predetermined degree of longitudinal foreshortening. In some embodiments the stent does not substantially longitudinally foreshorten when being expanded from an unexpanded state to an expanded state.

In at least one embodiment the stent comprises struts or other elements that are moveable in a longitudinal direction to a greater extent than elements in line with the push wire.

In at least on embodiment the stent is delivered into a body lumen through a catheter. In some embodiments, the stent is configured such that in the folded or collapsed state individual stent components resist tuliping thereby allowing the stent to pass through the catheter with reduced frictional interference.

These and other embodiments which characterize the invention are pointed out with particularity in the claims annexed hereto and forming a part hereof. However, for a better understanding of the invention, its advantages and objectives obtained by its use, reference should be made to the drawings which form a further part hereof and the accompanying descriptive matter, in which there is illustrated and described a embodiments of the invention.

A device as defined in the preamble of claim 1 is disclosed in WO 94/03127.

### BRIEF DESCRIPTION OF THE SEVERAL VIEWS OF THE DRAWING(S)

A detailed description of the invention is hereafter described with specific reference being made to the drawings.
FIG. 1 is a longitudinal side view of an embodiment of the invention in a predeployed configuration.
FIG. 2 is a longitudinal side view of the embodiment shown in FIG. 1 depicted during initial deployment.
FIG. 3 is a longitudinal side view of the embodiment shown in FIG. 1 in an initially deployed configuration.
FIG. 4 is a longitudinal side view of the embodiment shown in FIG. 1 in a fully deployed configuration.
FIG. 5 is a longitudinal side view of an embodiment of the invention depicting the assembly in a predeployed configuration.
FIG. 6 is a longitudinal side view of the assembly shown in FIG. 5 wherein the stent is shown being initially deployed from the catheter.
FIG. 7 is a longitudinal side view of the assembly shown in FIG. 6 wherein the stent is shown partially expanded and partially unexpanded during initial deployment from the catheter.
FIG. 8 is a longitudinal side view of the assembly shown in FIG. 7 wherein the stent is shown partially expanded and partially unexpanded during initial deployment from the catheter.
FIG. 9 is a longitudinal side view of the assembly shown in FIG. 8 wherein the stent is shown partially expanded and partially unexpanded during initial deployment from the catheter.
FIG. 10 is a longitudinal side view of the assembly shown in FIGs 5-9 wherein the assembly is shown in an initially deployed configuration wherein the stent is in a fully expanded state but still engaged to the push wire.
FIG. 11 is a longitudinal side view of the assembly shown in FIGs 5-10 wherein the assembly is shown in a fully deployed configuration wherein the stent is in a fully expanded state but has been disengaged from the push wire.
FIG. 12 is a longitudinal top down view of an embodiment of the invention, wherein the stent is shown in an expanded state.

### DETAILED DESCRIPTION OF THE INVENTION

While this invention may be embodied in many different forms, there are described in detail herein specific preferred embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

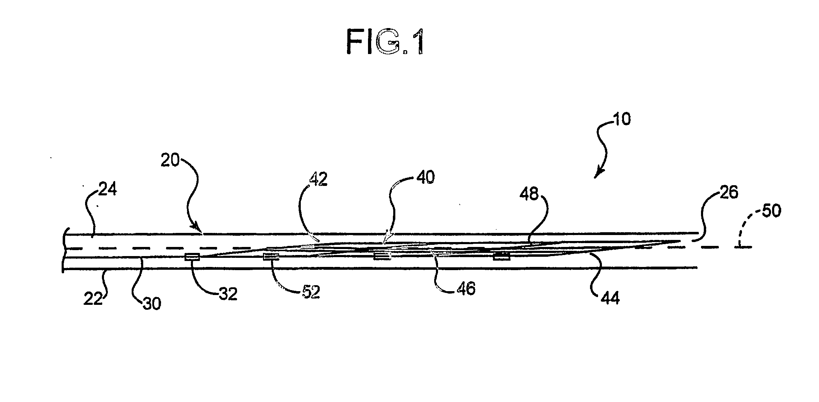

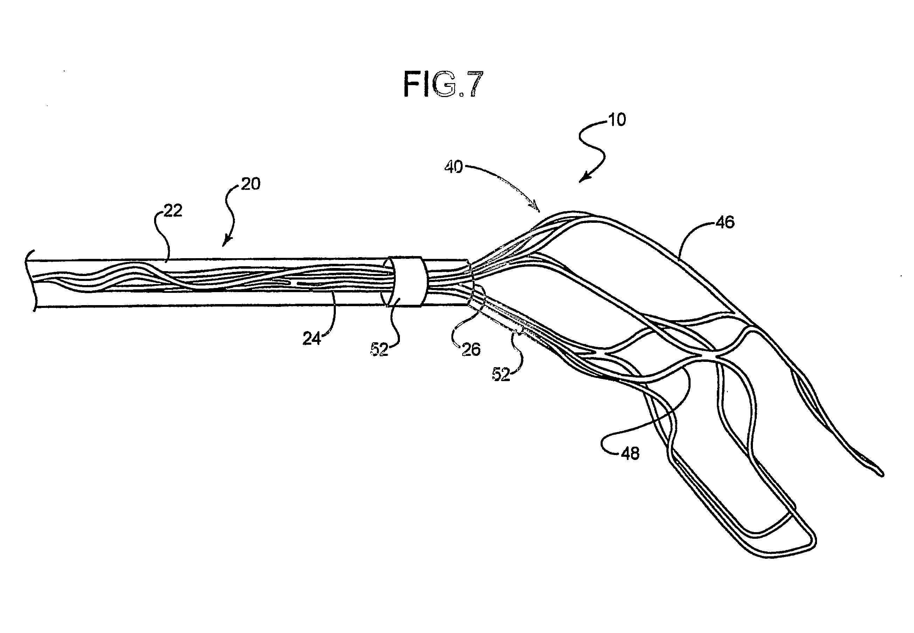

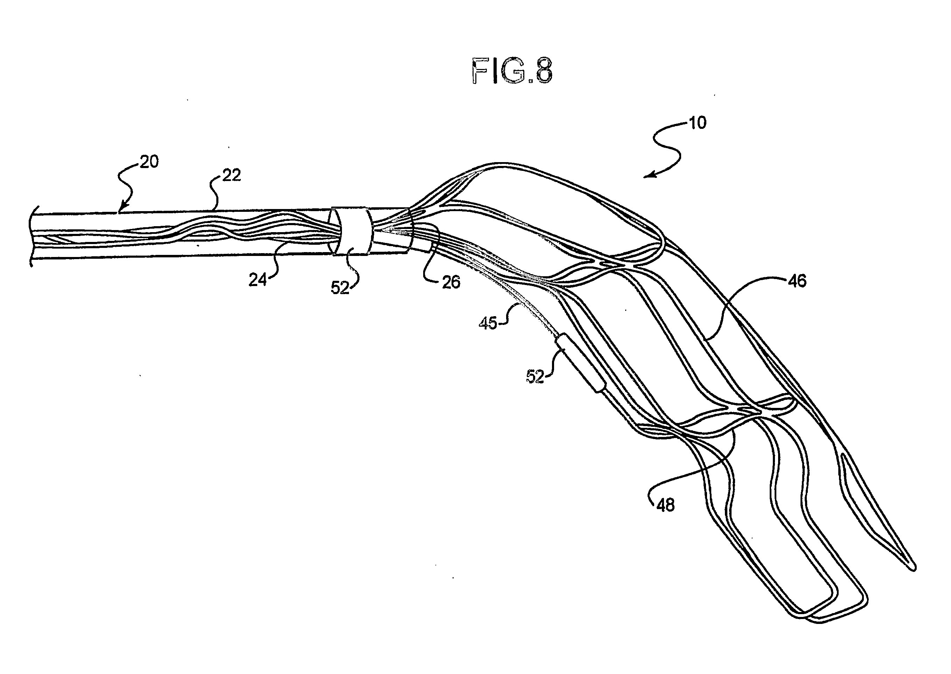

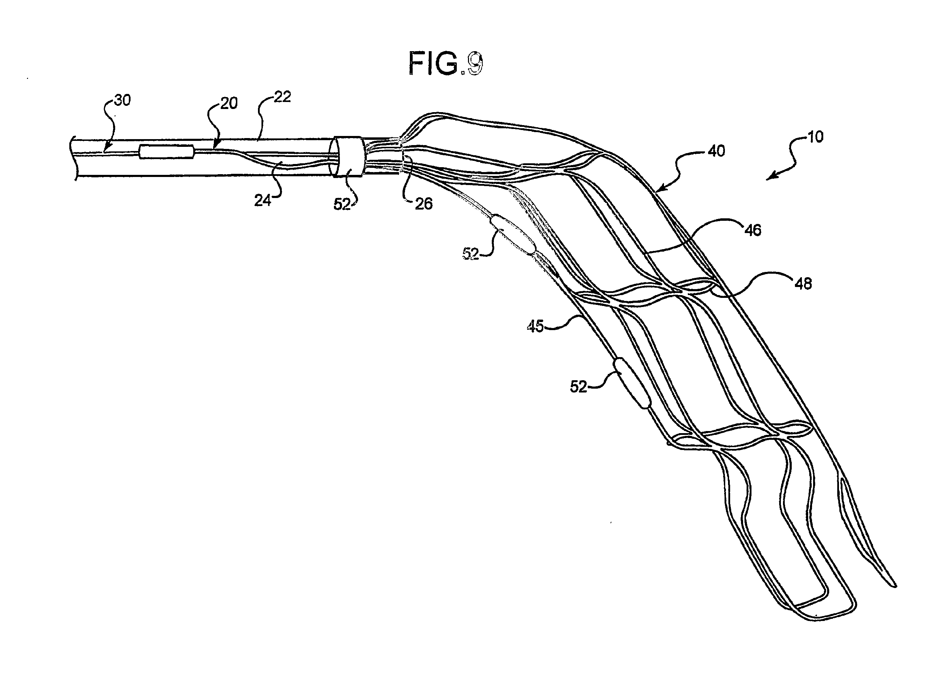

As indicated above the present invention is embodied in a variety of forms. Some examples of some embodiments are depicted in FIGs. 1-11.

In an embodiment of the invention depicted in FIGs. 1-11 an assembly 10 is depicted having a variety of components. The assembly 10 includes a catheter 20 a push wire 30 and an expandable stent 40.

Catheter 20 may be any type of catheter suitable for use in delivering a medical device to a body lumen. In at least one embodiment the catheter is a microcatheter such as may be suitable for use in delivering medical devices to intracranial spaces or vessels. An example of a suitable catheter 20 is the Excelsior TM microcatheter (1018) available from Boston Scientific Corporation of Natick, Massachusetts. Catheters such as those depicted in US 4739768, US 4979959, US 4973493, and US 5002582 are also suitable for use in the present invention. It is noted however that other catheter types and designs in addition to those described above may also be suitable for use as catheter 20.

Catheter 20 comprises a catheter shaft 22, which defines a lumen 24. The shaft 22 further defines an opening 26, which is in communication with the lumen 24 at the distal end 28 of the catheter 20.

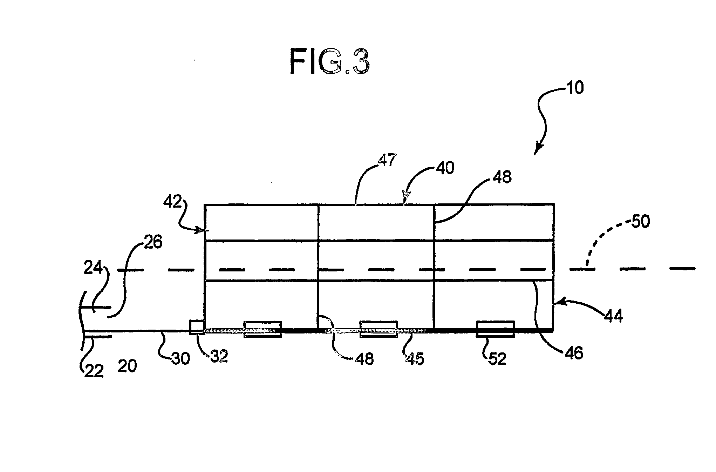

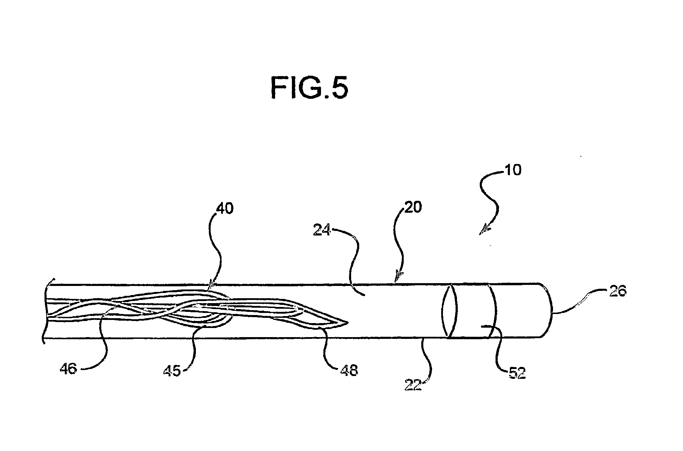

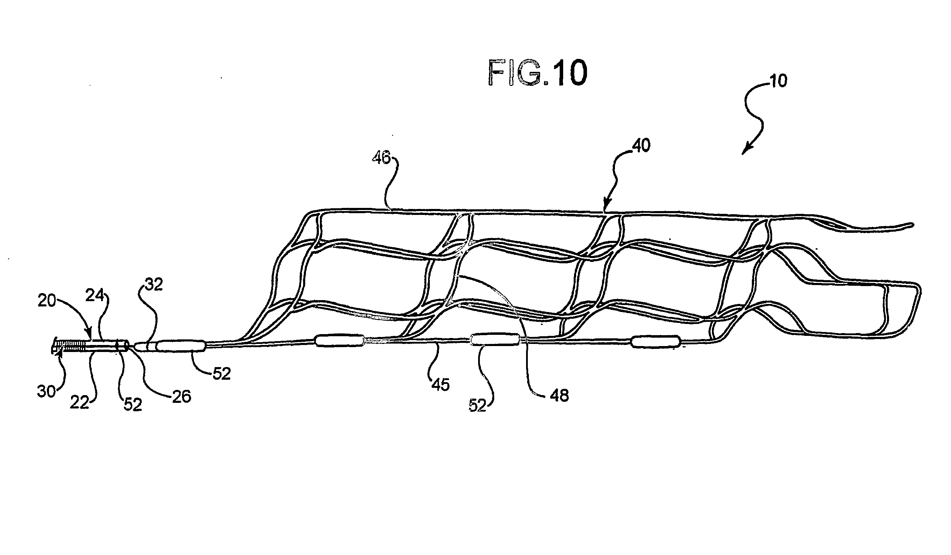

Stent 40 comprises a proximal end region 42 and a distal end region 44. The push wire 30 (not shown in FIG. 5-8) is removably engaged to the proximal end region 42 of the stent. As a result of the engagement of the push wire 30 to the stent 40, prior to fully deploying the stent 40 into a body lumen, the stent 40 may be readily advanced through the catheter lumen 24 by pushing the push wire 30 therethrough. Even after the stent 40 is fully expanded, such as is shown in FIGs. 3 and 10, the stent 40 may be repositioned within the lumen by advancing or withdrawing the push wire 30 to move the stent in a desired direction. In order to better facilitate advancement of the stent 40 through the catheter lumen 24 by pushing the push wire 30, the stent is provided with a unique backbone 45 which is in longitudinal alignment with the push wire 30 and provides greater column strength than the remaining individual components of the stent 40. When engaged to the stent 40 the push wire 30 effectively extends from the backbone 45 or vice versa.

In the embodiment shown in FIGs 1-11 the stent 40 comprises a backbone 45 and a plurality of first and second stent members or struts 46 and 48. When stent 40 is in the unexpanded state, such as is shown in FIG. 1 and 5, each of the first stent members 46 and second stent members 48 lay down along the backbone 45 such that members 46 and 48 are oriented in a substantially longitudinal direction. As is shown however in FIGs. 3-4 and 10-11 when the stent 40 is in the expanded state, the first members 46 remain oriented in a substantially longitudinal direction relative to the longitudinal axis 50 of the stent 40 while each of the second members 48 attain a substantially circumferential orientation.

The stent 40 may be self-expandable, inflation expandable, and/or hybrid expandable. Where the stent 40 is inflation expandable, or alternatively in some embodiments where the stent is hybrid expandable, the stent 40, in the unexpanded state, may be disposed about a balloon or other inflation mechanism which expands the stent after the stent is advanced out of the catheter 20.

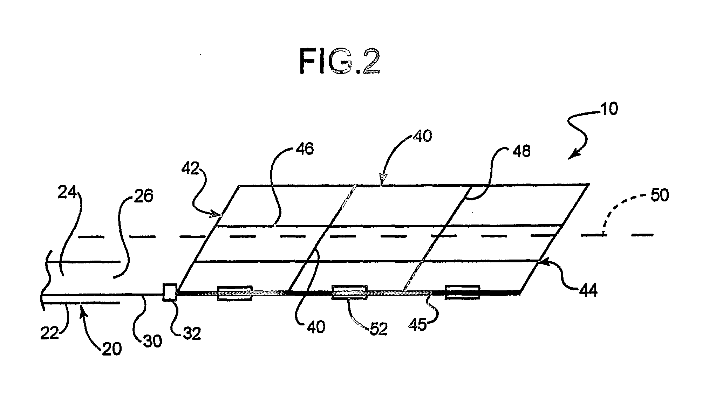

Where the stent 40 is a self-expandable stent the stent 40 is held in the unexpanded state by the catheter 20 in a predeployed configuration. When the stent 40 is advanced beyond the opening 26 of the catheter 20, or the stent 40 is held in place and the catheter 20 is withdrawn from about the stent 40, the stent 40 will begin to expand such as is shown in FIGs. 2 and 6-9. As is shown by comparing the various sates of expansion shown in FIGs 1-11, each of the first stent members 46 and each of the second stent members 48 expand relative to the backbone 45. As a result of this type of expansion, the stent 40 has a minimal degree of longitudinal foreshortening. In some embodiments the stent does not longitudinally foreshorten when expanded from the unexpanded state to the expanded state. In some embodiments the degree of longitudinal foreshortening between the unexpanded state and the expanded state is less than half of the circumference of the catheter opening 26. In some embodiments the degree of longitudinal foreshortening between the unexpanded state and the expanded state is less than 5% of the length of the stent.

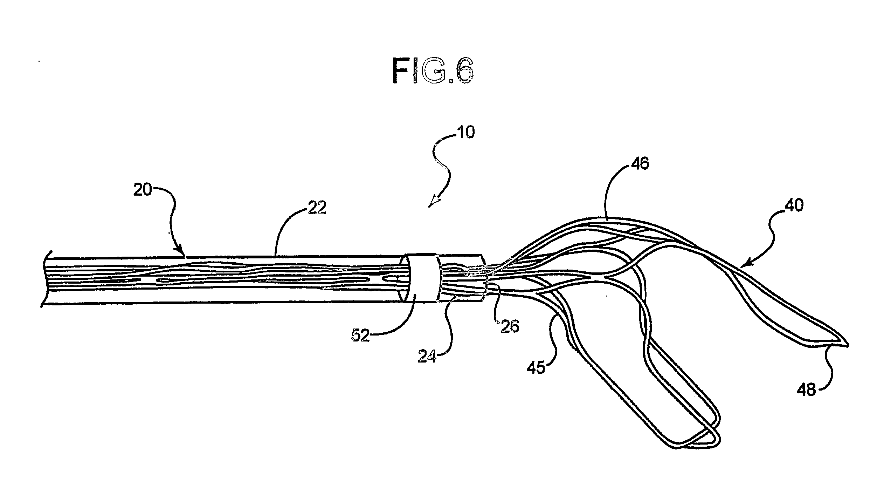

As is shown in FIG. 2, as well as in FIGs.6-9 , as the stent expands the second members 48 move from the more longitudinal orientation shown in FIGs. 1 and 5 to a more circumferential orientation or direction, such as is shown in FIGs 2-3 and 6-10. The second members 48 may be characterized as standing up' on the backbone 45. As a result of the second members 48 standing up on the backbone 45, the first members 46 positioned between adjacent second members 48 will be drawn radial away from the backbone 45 while remaining in a substantially longitudinal orientation relative thereto. In at least one embodiment, one or more of the first members remain; substantially parallel to the backbone 45 in the expanded state and the unexpanded state.

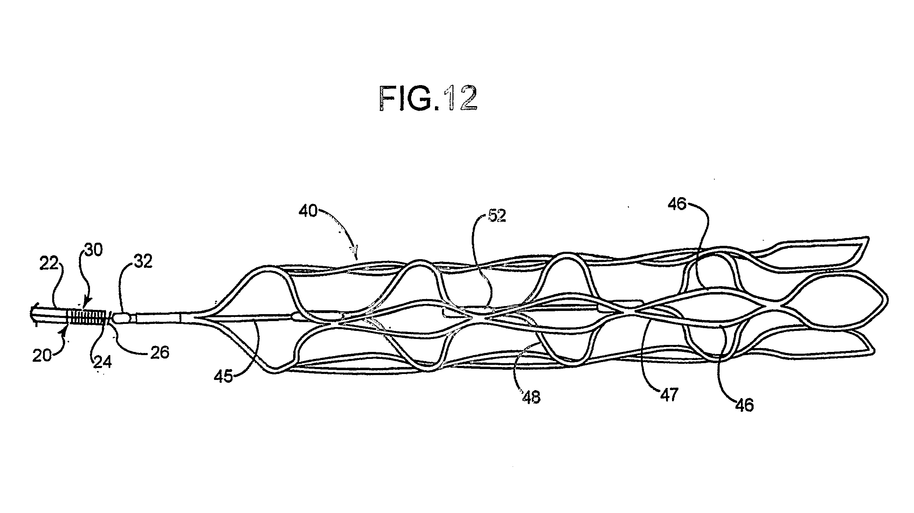

As indicated above the backbone 45 is longitudinally aligned with the push wire 30, and when the push wire 30 is engaged to the stent 40, the push wire extends from the backbone 45 or vice versa. In some embodiments the stent comprises a second backbone 47. The second backbone 47 may have an equal or different column ) strength to that of the first backbone 45. Backbones 45 and 47 may each be comprised of a single longitudinally oriented strut having a greater thickness than the thickness of the first or second members 46 and 48. Alternatively, one or more of the backbones 45 and 47 may be comprised of one or more first stent members 46 such as is best shown in FIG. 12. In some embodiments, in the unexpanded state the first backbone 45 is circumferentially and longitudinally offset from the second backbone 47, but in the expanded state the first backbone 45 is not longitudinally offset from the second backbone. In some embodiments, in the unexpanded state the first backbone 45 is circumferentially and longitudinally offset from the second backbone 47 to a predetermined degree but in the expanded state degree to which the first backbone 45 and the second backbone 47 are longitudinally offset is reduced.

Backbones 45 and 47 are typically substantially straight in configuration, tacking into account the need of the stent 40 to curve and bend within tortuous vessels or other body lumens. In some embodiments however, one or more of the backbones 45 and 47 may be configured to have one or more curved portions. However, regardless of the number and type of curved and/or straight portions within the first backbone 45, the first backbone 45 remains oriented in a substantially longitudinal direction in order to maintain pushability of the stent. In some embodiments the backbone 45 is parallel to the longitudinal axis 50 of the stent 40.

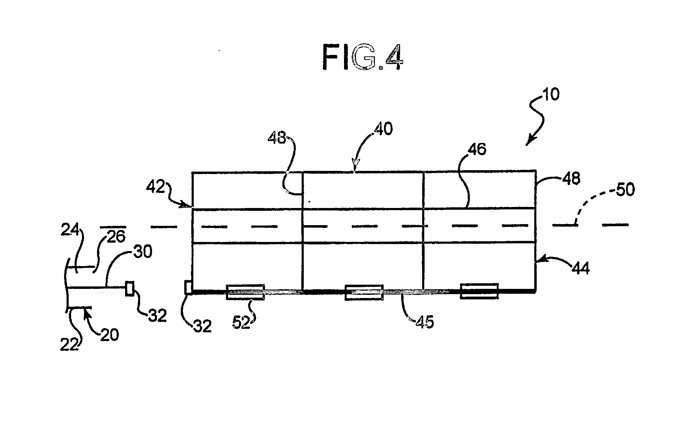

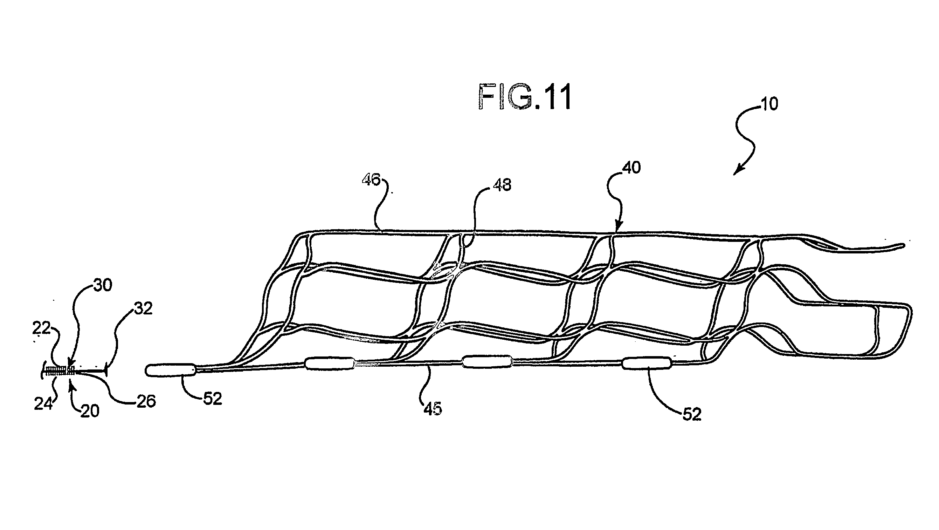

As indicated above, the push wire 30 is removably engaged to the stent 40. In some embodiments the push wire 30 is engaged to the stent at a severable junction 32. After the stent 40 has been advanced through the catheter 20, and the stent is expanded, such as is shown in FIGs. 3 and 6-9, the severable junction 32 is severed, such as is shown in FIG. 4 and FIG. 11 and the stent 40 is released from the push wire 30. In a fully deployed configuration such as is shown in FIGs. 4 and 11, the stent 40 is fully expanded and maintains its position in the lumen by frictional engagement with the lumen wall, the catheter 20 and guide wire 30 may be withdrawn from the lumen following full deployment of the stent 40.

Prior to severance of the severable junction 32 but following release of the stent 40 from the catheter 20 the assembly 10 is in the initially deployed configuration such as is shown in FIG. 10. In this configuration the stent 40 may be moved in a longitudinally in a proximal or distal direction as a result of the continued engagement to the guide wire 30. Even when the stent 40 is in the expanded state the stent may be manipulated in this manner. Such continued engagement with the push wire 30 allows the stent 40 to be retracted back into the catheter lumen 24 if it is desired to significantly reposition the stent 40 or abort the deployment entirely.

Junction 32 may be severed using any of a variety of different methods including, but not limited to, bioabsorption, electrolytic corrosion, mechanical actuation, hydraulic pressure, thermal processes, electromagnetic energy, and so forth as described above. Other methods of detachment known to those of skill in the art but not described herein may also be employed in releasing the device of the present invention.

Some examples of severable junctions which may be employed in the present invention are described, for example, in US 5122136; US 5354295; US 5540680; US 5855578; US 5895385; US 5925037; US 5944714; US 5947963; US 5976126; US 6010498; US 6066133; US 6083220; US Pat. Application No. 10/230803, filed August 29, 2002, and entitled *Device for Closure of a Vascular Defect and Method for Treating Same;* US Pat. Application No. 10/231391, filed August 29, 2002 and entitled *Device and Method for Treatment of a Vascular Defect.*

In some embodiments of the invention one or more components of the assembly 10, including the catheter 20, push wire 30 and/or stent 40 may be at least partially radiopaque. In some embodiments, the backbone 45 of the stent 40 has one or more radiopaque markers 52 engaged thereto. Markers 52 may comprise a coating of radiopaque material, a radiopaque band or fastener engaged to the backbone 45 or any other radiopaque mechanism suitable for use in a stent delivery system. In some embodiments such as is best shown in FIG. 5 a portion of the catheter 20 may be equipped with a radiopaque marker 52 adjacent to the opening 26, however one or more markers may be positioned anywhere desired on the catheter 20 or other assembly component.

As is known in the art stents may have a variety of configuration, methods of manufacture, materials, etc. In the present invention the stent 40 may be at least partially constructed from a suitable stent material including but not limited to: stainless steel, Elgiloy, nickel, titanium, and alloys thereof. Other materials include shape memory polymers and shape memory metals such as nitinol.

In some embodiments the stent 40 may be constructed by cutting the desired stent pattern of a stent 40 having one or more backbones 45 and 47 and a plurality of first stent members 46 and second stent members 48 as described above, from a tube of suitable stent material.

In some embodiments the stent 40 may be constructed from one or more wires of suitable stent material, wherein at least one wire is arranged to form a stent 40 having one or more backbones 45 and 47 and a plurality of first stent members 46 and second stent members 48 as described above.

In some embodiments the stent 40 is constructed in accordance with any desired or known construction technique. The backbone 45 may then be formed by masking the area of the stent which corresponds to the position of the backbone. The unmasked portion of the stent is then microblasted, electropolished, and/or otherwise processed to reduce the thickness of the unmasked portion of the stent while maintaining the thickness of the masked portion. Following such processing the masking is removed and the stent 40 is provided with a backbone 45 with a greater columnar strength than the remaining portions of the stent.

The various embodiments of the stents described herein may include one or more coatings and/or other delivery mechanisms which comprise one or more therapeutic agents, cellular materials, polymeric agents, drugs, etc.

The therapeutic agent may be non-genetic or genetic. Suitable non-genetic therapeutic agents include anti-thrombogenic agents such as heparin, heparin derivatives, urokinase, and PPack (dextrophenylalanine proline arginine chloromethylketone), anti-proliferative agents such as enoxaprin, angiopeptin, or monoclonal antibodies capable of blocking smooth muscle cell proliferation, hirudin, and acetylsalicylic acid, anti-inflammatory agents such as dexamethasone, prednisolone, corticosterone, budesonide, estrogen, sulfasalazine, and mesalamine, antineoplastic/antiproliferative/anti-miotic agents such as paclitaxel, 5-fluorouracil, cisplatin, vinblastine, vincristine, epothilones, endostatin, angiostatin and thymidine kinase inhibitors, anesthetic agents such as lidocaine, bupivacaine, and ropivacaine, anti-coagulants such as D-Phe-Pro-Arg chloromethyl keton, an RGD peptide-containing compound, heparin, antithrombin compounds, platelet receptor antagonists, antithrombin antibodies, anti-platelet receptor antibodies, aspirin, prostaglandin inhibitors, platelet inhibitors and tick antiplatelet peptides, vascular cell growth promoters such as growth factor inhibitors, growth factor receptor antagonists, transcriptional activators, and translational promoters, vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin, cholesterol-lowering agents; vasodilating agents; and agents which interfere with endogenous vascoactive mechanisms.

Suitable genetic materials include anti-sense DNA and RNA, DNA coding for anti-sense RNA, tRNA or rRNA to replace defective or deficient endogenous molecules, angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin like growth factor, cell cycle inhibitors including CD inhibitors, thymidine kinase ("TK") and other agents useful for interfering with cell proliferation, the family of bone morphogenic proteins ("BMP's"), BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Any of BMP-2, BMP-3, BMP-4, BMP-5, BMP-6 and BMP-7 are particularly desirable. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the "hedgehog" proteins, or the DNA's encoding them.

Suitable cellular materials include cells of human origin (autologous or allogeneic) or from an animal source (xenogeneic), genetically engineered if desired to deliver proteins of interest at the transplant site. The delivery media can be formulated as needed to maintain cell function and viability.

Suitable polymer coating materials include polycarboxylic acids, cellulosic polymers, including cellulose acetate and cellulose nitrate, gelatin, polyvinylpyrrolidone, cross-linked polyvinylpyrrolidone, polyanhydrides including maleic anhydride polymers, polyamides, polyvinyl alcohols, copolymers of vinyl monomers such as EVA, polyvinyl ethers, polyvinyl aromatics, polyethylene oxides, glycosaminoglycans, polysaccharides, polyesters including polyethylene terephthalate, polyacrylamides, polyethers, polyether sulfone, polycarbonate, polyalkylenes including polypropylene, polyethylene and high molecular weight polyethylene, halogenated polyalkylenes including polytetrafluoroethylene, polyurethanes, polyorthoesters, proteins, polypeptides, silicones, siloxane polymers, polylactic acid, polyglycolic acid, polycaprolactone, polyhydroxybutyrate valerate and blends and copolymers thereof, coatings from polymer dispersions such as polyurethane dispersions (BAYHDROL^{®}, etc.), fibrin, collagen and derivatives thereof, polysaccharides such as celluloses, starches, dextrans, alginates and derivatives, hyaluronic acid, squalene emulsions. Desirably, polyacrylic acid, available as HYDROPLUS^{®} (Boston Scientific Corporation, Natick, Mass.), and described in U.S. Pat. No. 5,091,205, the disclosure of which is hereby incorporated herein by reference, may be used. Also desirably, the polymer may be a copolymer of polylactic acid and polycaprolactone. Other materials include selected medical-grade biodegradable materials such as PGA-TMC, Tyrosine-Derived Polycarbonates and arylates, polycaprolactone co butyl acrylate and other co polymers, Poly-L-lactic acid blends with DL-Lactic Acid, Poly(lactic acid-co-glycolic acid), polycaprolactone co PLA, polycaprolactone co butyl acrylate and other copolymers, Tyrosine-Derived Polycarbonates and arylate, poly amino acid, polyphosphazenes, polyiminocarbonates, polydimethyltrimethylcarbonates, biodegradable CA/PO₄'s, cyanoacrylate, 50/50 DLPLG, polydioxanone, polypropylene fumarate, or polydepsipeptides.

Other suitable coatings include macromolecules such as chitosan and Hydroxylpropylmethylcellulose. Surface erodible materials may also be used. Coatings may also comprise maleic anhydride copolymers, zinc-calcium phosphate and amorphous polyanhydrides.

In some embodiments the stent or one or more portions thereof may be provided with a hydrophilic and/or a hydrophobic coating.

The inventive medical devices may also be provided with a sugar or more generally a carbohydrate and/or a gelatin to maintain the inventive medical devices on a balloon during delivery of the medical device to a desired bodily location. Other suitable compounds for treating the inventive medical devices include biodegradable polymers and polymers which are dissolvable in bodily fluids. Portions of the interior and/or exterior of the inventive medical devices may be coated or impregnated with the compound. Mechanical retention devices may also be used to maintain the inventive medical devices on the balloon during delivery.

The inventive medical devices may also be provided in whole or in part with one or more of the above therapeutic agents, polymeric coatings or the like. Where multiple therapeutic agents are provided, different coatings and/or mechanisms may release the drugs at different rates. For example, one therapeutic agent may be released at a fast rate and another therapeutic agent may be released at a slow rate. Where multiple polymeric coatings are provided, the coatings may degrade or erode at different rates. In order to facilitate the retention and delivery of one or more therapeutic agents any of the stent embodiments described herein may be provided with a plurality of cavities, micro holes, slits, and/or other surface features such as are known in the art. Such surface features increase or otherwise alter the surface area of the stent to provide the stent with a more optimum agent delivery mechanism. Where the stent is provided with one or more cavities, the cavities may extend partially or entirely through the width of a given stent component. Any of the components of the stent may be provided with one or more cavities.

The above disclosure is intended to be illustrative and not exhaustive. This description will suggest many variations and alternatives to one of ordinary skill in this art. All these alternatives and variations are intended to be included within the scope of the claims where the term "comprising" means "including, but not limited to". Those familiar with the art may recognize other equivalents to the specific embodiments described herein which equivalents are also intended to be encompassed by the claims.

## Claims

1. A stent assembly (10) comprising a stent (40) the stent (40) having a proximal end (42) and a distal end (44) and being configurable between an unexpanded state and an expanded state, the stent (40) comprising
a plurality of interconnected first stent members (46) and second stent members (48), each of the first stent members (46) being oriented in a substantially longitudinal direction in the unexpanded state and the expanded state, each of the second stent members (48) being oriented in a substantially longitudinal direction in the unexpanded state and being oriented in a substantially circumferential direction in the expanded state, **characterised by** a first stent backbone (45) which extends from the proximal end (42) of the stent (40) to the distal (44) end of the stent, the first stent back bone (46) being oriented in a direction which is substantially parallel to a longitudinal axis of the stent (40), wherein the first stent backbone (45) having a greater column strength than the plurality of interconnected stent members (46, 48).

2. The assembly (10) of claim 1 wherein the first stent backbone (45) has a predetermined thickness and each of the plurality of interconnected first stent members (46) and second stent members (48) have a predetermined thickness, the predetermined thickness of the first stent backbone (45) being greater than the predetermined thickness of each of the plurality of interconnected first stent members (46) and second stent members (48).

3. The assembly (10) of claim 1 wherein further comprising a second stent backbone (47), at least a portion of the second stent backbone (47) being substantially parallel to the longitudinal axis of the stent (40) in the unexpanded state and the expanded state.

4. The assembly (10) of claim 3 wherein the second backbone is comprised of at least two longitudinally adjacent first stent members.

5. The assembly (10) of claim1 further comprising a push wire (30), the push wire (30) having a proximal end and a distal end, the distal end of the push wire being (30) removeably engaged to a proximal end (42) of the stent (40), the first backbone (45) extending from the distal end of the push wire (30).

6. The assembly (10) of claim 5 wherein the push wire (30) is removeably engaged to the stent (40) at a severable junction (32), the stent (40) being released from the push (30) wire when the severable junction (32) is severed.

7. The assembly (10) of claim 6 wherein at least a portion of the severable junction (32) is bioabsorbable, the stent (40) being released from the push wire (30) when the at least a portion of the severable junction (32) is bioabsorbed.

8. The assembly (10) of claim 6 wherein the severable junction (32) is constructed and arranged to be severable by at least one mechanism selected from the group consisting of : electrolytic corrosion, mechanical actuation, application of hydraulic pressure, application of thermal energy, application of electromagnetic energy and any combination thereof.

9. The assembly (10) of claim 5 comprising a predeployed configuration, an initially deployed configuration and a fully deployed configuration, in the predeployed configuration the stent (40) in the unexpanded state being in mechanical communication with the push wire (30), in the initially deployed configuration the stent (40) in the expanded state is in mechanical communication with the push wire (30), in the deployed configuration the stent (40) in the expanded state is mechanically independent from the push wire (30).

10. The assembly (10) of claim 9 wherein the assembly is constructed and arranged to be configurable from the predeployed configuration to the initially deployed configuration and from the initially deployed configuration to the fully deployed configuration.

11. The assembly (10) of claim 10 further comprising a catheter (20), the catheter comprising a catheter shaft (22), the catheter shaft (22) defining a lumen (24), the shaft (22) further defining an opening (26) at a distal end (28) of the catheter, in the predeployed configuration the stent (40) and push wire (30) being moveably contained within the lumen (24).

12. The assembly (10) of claim 11 wherein when the assembly is configured from the predeployed configuration to the initially deployed configuration at least a portion of the push wire (30) and the stent (40) are advanced through the opening (26) at the distal end (28) of the catheter (20).

## Patentansprüche

1. Stentanordnung (10), umfassend einen Stent (40), wobei der Stent (40) ein proximales Ende (42) und ein distales Ende (44) aufweist und zwischen einem nicht entfalteten Zustand und einem entfalteten Zustand konfigurierbar ist, wobei der Stent (40) umfasst:
eine Mehrzahl miteinander verbundener erster Stentelemente (46) und zweiter Stentelemente (48), wobei jedes der ersten Stentelemente (46) im nicht entfalteten Zustand und im entfalteten Zustand im Wesentlichen in Längsrichtung ausgerichtet ist, wobei jedes der zweiten Stentelemente (48) im nicht entfalteten Zustand im Wesentlichen in Längsrichtung ausgerichtet ist und im entfalteten Zustand im Wesentlichen in Umfangsrichtung ausgerichtet ist, **gekennzeichnet durch**
eine erste Stent-Hauptstrebe (45), die sich vom proximalen Ende (42) des Stents (40) bis zum distalen Ende (44) des Stents erstreckt, wobei die erste Stent-Hauptstrebe (45) in einer Richtung ausgerichtet ist, die im Wesentlichen parallel zu einer Längsachse des Stents (40) verläuft, wobei die erste Stent-Hauptstrebe (45) eine größerer Säulenfestigkeit aufweist als die Mehrzahl der miteinander verbundenen Stentelemente (46, 48).

2. Baugruppe (10) nach Anspruch 1, wobei die erste Stent-Hauptstrebe (45) eine vorbestimmte Stärke aufweist und jedes der Mehrzahl miteinander verbundener erster Stentelemente (46) und zweiter Stentelemente (48) eine vorbestimmte Dicke aufweist, wobei die vorbestimmte Dicke der ersten Stent-Hauptstrebe (45) größer ist als die vorbestimmte Dicke jedes der Mehrzahl miteinander verbundener erster Stentelemente (46) und zweiter Stentelemente (48).

3. Baugruppe (10) nach Anspruch 1, wobei sie überdies eine zweite Stent-Hauptstrebe (47) umfasst, wobei mindestens ein Abschnitt der zweiten Stent-Hauptstrebe (47) im nicht entfalteten Zustand und im entfalteten Zustand im Wesentlichen parallel zur Längsachse des Stents (40) verläuft.

4. Baugruppe (10) nach Anspruch 3, wobei die zweite Hauptstrebe mindestens aus zwei in Längsrichtung verlaufenden benachbarten ersten Stentelementen besteht.

5. Baugruppe (10) nach Anspruch 1, überdies umfassend einen Schiebedraht (30), wobei der Schiebedraht (30) ein proximales Ende und ein distales Ende aufweist, wobei das distale Ende des Schiebedrahts (30) abnehmbar mit einem proximalen Ende (42) des Stents (40) in Eingriff ist, wobei sich die erste Hauptstrebe (45) vom distalen Ende des Schiebedrahts (30) erstreckt.

6. Baugruppe (10) nach Anspruch 5, wobei der Schiebedraht (30) abnehmbar mit dem Stent (40) an einer trennbaren Verbindung (32) in Eingriff ist und der Stent (40) von dem Schiebedraht (30) gelöst ist, wenn die trennbare Verbindung (32) getrennt wird.

7. Baugruppe (10) nach Anspruch 6, wobei mindestens ein Abschnitt der trennbaren Verbindung (32) bioabsorbierbar ist, wobei der Stent (40) von dem Schiebedraht (30) gelöst wird, wenn mindestens ein Abschnitt der bioabsorbierbaren Verbindung (32) bioabsorbierbar ist.

8. Baugruppe (10) nach Anspruch 6, wobei die trennbare Verbindung (32) so gebaut und angeordnet ist, dass sie durch mindestens einen Mechanismus trennbar ist, der aus der Gruppe ausgewählt wird, die besteht aus: Elektrolytischer Korrosion, mechanischer Betätigung, Anwendung von hydraulischem Druck, Anwendung von Wärmeenergie, Anwendung von elektromagnetischer Energie und eine beliebige Kombination daraus.

9. Baugruppe (10) nach Anspruch 5, umfassend eine zuvor eingesetzte Konfiguration , eine anfänglich eingesetzte Konfiguration und eine vollständig eingesetzte Konfiguration, wobei der Stent (40) im nicht entfalteten Zustand in der zuvor eingesetzten Konfiguration in mechanischer Kommunikation mit dem Schiebedraht (30) steht, der Stent (40) im entfalteten Zustand in der anfänglich eingesetzten Konfiguration in mechanischer Kommunikation mit dem Schiebedraht (30) steht, der Stent (40) im entfalteten Zustand in der eingesetzten Konfiguration mechanisch unabhängig von dem Schiebedraht (30) ist.

10. Baugruppe (10) nach Anspruch 9, wobei die Baugruppe so gebaut und angeordnet ist, dass sie von der zuvor eingesetzten Konfiguration bis zur anfänglich eingesetzten Konfiguration und von der anfänglich eingesetzten Konfiguration zur vollständig eingesetzten Konfiguration konfigurierbar ist.

11. Baugruppe (10) nach Anspruch 10, überdies umfassend einen Katheter (20), wobei der Katheter einen Katheterschaft (22) umfasst, wobei der Katheterschaft (22) ein Lumen (24) bestimmt, wobei der Schaft (23) überdies eine Öffnung (26) am distalen Ende (28) des Katheters bestimmt, wobei der Stent (40) und der Schiebedraht (30) in der zuvor eingesetzten Konfiguration innerhalb des Lumens (24) beweglich enthalten sind.

12. Baugruppe (10) nach Anspruch 11, wobei, wenn die Baugruppe von der zuvor eingesetzten Konfiguration bis zur anfänglich eingesetzten Konfiguration konfiguriert wird, mindestens ein Abschnitt des Schiebedrahts (30) und der Stent (40) durch die Öffnung (26) an dem distalen Ende (28) des Katheters (20) vorgeschoben werden.

## Revendications

1. Ensemble drain tuteur (10), comprenant un drain tuteur (40), le drain tuteur (40) présentant une extrémité proximale (42) et une extrémité distale (44) et étant configurable entre un état non-expansé et un état expansé, le drain tuteur (40) comprenant
une multiplicité de premiers éléments (46) de drain tuteur et seconds éléments (48) de drain tuteur interconnectés, chacun des premiers éléments (46) de drain tuteur étant orienté dans une direction essentiellement longitudinale dans l'état non expansé et l'état expansé, chacun des seconds éléments (48) de drain tuteur étant orienté dans une direction essentiellement longitudinale dans l'état non-expansé et étant orienté dans une direction essentiellement circonférentielle dans l'état expansé,
**caractérisé par**
une première épine dorsale (45) de drain tuteur qui s'étend depuis l'extrémité proximale (42) du drain tuteur (40) à l'extrémité distale (44) du drain tuteur, la première épine dorsale (45) de drain tuteur étant orientée dans une direction qui est essentiellement parallèle à un axe longitudinal du drain tuteur (40), la première épine dorsale (45) présentant une résistance au flambage plus élevée que la multiplicité d'éléments (46, 48) de drain tuteur interconnectés.

2. Ensemble (10) selon la revendication 1, la première épine dorsale (45) de drain tuteur ayant une épaisseur prédéfinie et chacun de la multiplicité de premiers éléments (46) de drain tuteur et de seconds éléments (48) de drain tuteur interconnectés ayant une épaisseur prédéfinie, l'épaisseur prédéfinie de la première épine dorsale (45) de drain tuteur étant plus grande que l'épaisseur prédéfinie de chacun de la multiplicité de premiers éléments (46) de drain tuteur et de seconds éléments (48) de drain tuteur interconnectés.

3. Ensemble (10) selon la revendication 1, comprenant en outre une seconde épine dorsale (47) de drain tuteur, au moins une partie de la seconde épine dorsale (47) de drain tuteur étant essentiellement parallèle à l'axe longitudinal du drain tuteur (40) dans l'état non-expansé et dans l'état expansé.

4. Ensemble (10) selon la revendication 3, la seconde épine dorsale comprenant au moins deux premiers éléments de drain tuteur adjacents longitudinalement.

5. Ensemble (10) selon la revendication 1, comprenant en outre un fil à pousser (30), le fil à pousser (30) présentant une extrémité proximale et une extrémité distale, l'extrémité distale du fil à pousser (30) étant en prise de manière détachable avec une extrémité proximale (42) du drain tuteur (40), la première épine dorsale (45) s'étendant depuis l'extrémité distale du fil à pousser (30).

6. Ensemble (10) selon la revendication 5, le fil à pousser (30) étant en prise de manière détachable avec le drain tuteur (40) à un raccordement séparable (32), le drain tuteur (40) étant libéré du fil à pousser (30) lorsque le raccordement séparable (32) est séparé.

7. Ensemble (10) selon la revendication 6, au moins une partie du raccordement séparable (32) étant bioabsorbable, le drain tuteur (40) étant libéré du fil à pousser (30) lorsque la au moins une partie du raccordement séparable (32) est bioabsorbée.

8. Ensemble (10) selon la revendication 6, le raccordement séparable (32) étant construit et arrangé pour être séparable par au moins un mécanisme choisi dans le groupe consistant en : corrosion électrolytique, actionnement mécanique, application de pression hydraulique, application d'énergie calorifique, application d'énergie électromagnétique et toute combinaison de ceux-ci.

9. Ensemble (10) selon la revendication 5, comprenant une configuration pré-déployée, une configuration initialement déployée et une configuration complètement déployée, le drain tuteur (40) dans l'état non-expansé étant dans la configuration pré-déployée en communication mécanique avec le fil à pousser (30), le drain tuteur (40) dans l'état expansé est dans la configuration initialement déployée en communication mécanique avec le fil à pousser (30), le drain tuteur (40) dans l'état expansé est dans la configuration déployée mécaniquement indépendant du fil à pousser (30).

10. Ensemble (10) selon la revendication 9, l'ensemble étant construit et agencé pour être configurable de la configuration pré-déployée à la configuration initialement déployée et de la configuration initialement déployée à la configuration complètement déployée.

11. Ensemble (10) selon la revendication 10, comprenant en outre un cathéter (20), le cathéter comprenant une tige (22) de cathéter, la tige (22) de cathéter définissant une lumière (24), la tige (22) définissant en outre une ouverture (26) à une extrémité distale (28) du cathéter, dans la configuration pré-déployée, le drain tuteur (40) et le fil à pousser (30) étant contenus de manière mobile dans la lumière (24).

12. Ensemble (10) selon la revendication 11, dans lequel lorsque l'ensemble est configuré de la configuration pré-déployée à la configuration initialement déployée, au moins une partie du fil à pousser (30) et le drain tuteur (40) sont avancés à travers l'ouverture (26) à l'extrémité distale (28) du cathéter (20).
